# FASCICULE DE BREVET EUROPEEN

(11) **EP 2 430 171 B1**
(45) Date de publication et mention de la délivrance du brevet: **01.04.2015**
(21) Numéro de dépôt: 10723169.8
(22) Date de dépôt: 06.05.2010
(51) Int. Cl.: C12P 7/10, C12P 7/28, C12P 7/16

(54) **PROCÉDÉ DE PRODUCTION D'ALCOOLS ET/OU DE SOLVANTS À PARTIR DE BIOMASSE LIGNOCELLULOSIQUE AVEC RECYCLAGE ACIDE DES RÉSIDUS SOLIDES**
VERFAHREN ZUR HERSTELLUNG VON ALKOHOLEN UND/ODER LÖSUNGSMITTELN AUS LIGNOCELLULOSE-BIOMASSE MIT SÄURE-RECYCLING FESTER RÜCKSTÄNDE
METHOD FOR PRODUCING ALCOHOLS AND/OR SOLVENTS FROM LIGNOCELLULOSIC BIOMASS WITH ACID RECYCLING OF SOLID RESIDUES

(30) Priorité: 15.05.2009 FR 0902347
(43) Date de publication de la demande: 21.03.2012
(73) Titulaire: IFP Energies nouvelles, 92852 Rueil-Malmaison Cedex (FR)
(72) Inventeur: ROPARS, Marcel, F-91120 Palaiseau (FR); AYMARD, Caroline, F-69003 Lyon (FR)
(86) Numéro de dépôt international: PCT/FR2010/000350
(87) Numéro de publication internationale: WO 2010/130888

(56) Documents cités:
- WO-A1-2004/113549
- WO-A1-2008/017145
- WO-A1-2009/004273
- FR-A1- 2 903 119
- CARVALHEIRO, FLORBELA ET AL.: "Hemicellulose biorefineries: a review on biomass pretreatments", JOURNAL OF SCIENTIFIC AND INDUSTRIAL RESEARCH, vol. 67, no. 11, 1 novembre 2008 (2008-11-01), pages 849-864, XP002568985, ISSN: 0022-4456

## Description

### DOMAINE DE L'INVENTION

La présente invention s'inscrit dans le cadre d'un procédé de production d'alcools et/ou de solvants dit de "seconde génération" à partir de biomasse lignocellulosique. Elle concerne plus particulièrement un procédé de production d'éthanol et/ou d'un mélange Acétone-Butanol-Ethanol (encore appelé mélange ABE).

### ART ANTÉRIEUR

La biomasse lignocellulosique représente une des ressources renouvelables les plus abondantes sur terre. Les substrats considérés sont très variés, puisqu'ils concernent à la fois les substrats ligneux (feuillus et résineux), les sous-produits de l'agriculture (paille) ou ceux des industries génératrices de déchets lignocellulosiques (industries agroalimentaires, papeteries).

La biomasse lignocellulosique est composée de trois principaux polymères : la cellulose (35 à 50%), l'hémicellulose (20 à 30%) qui est un polysaccharide essentiellement constitué de pentoses et d'hexoses et la lignine (15 à 25%) qui est un polymère de structure complexe et de haut poids moléculaire, composé d'alcools aromatiques reliés par des liaisons éther.

Ces différentes molécules sont responsables des propriétés intrinsèques de la paroi végétale et s'organisent en un enchevêtrement complexe.

La cellulose et éventuellement les hémicelluloses sont les cibles de l'hydrolyse enzymatique mais ne sont pas directement accessibles aux enzymes. C'est la raison pour laquelle ces substrats doivent subir un prétraitement précédant l'étape d'hydrolyse enzymatique. Le prétraitement vise à modifier les propriétés physiques et physico-chimiques du matériau lignocellulosique, en vue d'améliorer l'accessibilité de la cellulose emprisonnée au sein de la matrice de lignine et d'hémicellulose.

De nombreuses technologies pour réaliser ce prétraitement existent : cuissons acides, cuissons alcalines, explosion à la vapeur, procédés organosolv, etc. L'efficacité du prétraitement se mesure à la fois par le bilan matière à l'issue du prétraitement (taux de récupération des sucres sous forme monomère ou oligomère solubles ou polymère solide) et également par la susceptibilité à l'hydrolyse des résidus cellulosiques et hémicellulosiques.

Les prétraitements en conditions acides tendent à former des produits de dégradations des sucres pentoses et hexoses (par exemple le furfural, le 5-HMF) ainsi que de nombreux autres produits tels que des acides organiques, des aldéhydes ou alcools phénoliques, eux aussi issus des dégradations acides des sucres et de la lignine partiellement solubilisée. Ces produits de dégradation peuvent inhiber les organismes fermentaires selon leur concentration. La formation de ces produits de dégradation augmente avec la sévérité du prétraitement (chaleur, temps de rétention, acidité). L'hémicellulose du substrat lignocellulosique est très facilement hydrolysée en conditions acides et à haute température. Néanmoins, un prétraitement trop peu sévère risque de ne pas agir suffisamment sur la lignine présente et donc de diminuer la susceptibilité du substrat à l'hydrolyse enzymatique.

Les prétraitements en milieux alcalins ont l'avantage de ne pas générer de produits de dégradations. Ils présentent une alternative viable aux prétraitements acides, bien que leur coût, notamment pour les produits chimiques mis en oeuvre, soit plus élevé aujourd'hui.

A l'heure actuelle, aucun prétraitement, acide, alcalin ou autre, n'apparaît comme leader sur le plan technico-économique (Eggeman et al. Bioresource Technology 96 (2005) 2019-2025).

En vue de la conversion du substrat en alcools et/ou solvants, une hydrolyse enzymatique est menée sur le substrat prétraité. Les fractions lignine, cellulose, hémicellulose, présentes dans le substrat dépendent du prétraitement mis en oeuvre. Pour les prétraitements alcalins, le solide est constitué majoritairement de cellulose et d'hémicelluloses.

L'hydrolyse enzymatique se déroule à pH compris entre 4,5 et 5,5 et préférentiellement entre 4,8 et 5,2. La charge issue d'un prétraitement alcalin doit donc être neutralisée et ramenée au pH correct avant son entrée en hydrolyse enzymatique.

L'hydrolyse enzymatique est menée avec une solution enzymatique, souvent produite à partir du champignon filamenteux comme par exemple *Trichoderma reeseii,* ou parfois *Aspergillus niger.* Ces champignons sécrètent un "cocktail enzymatique" composés de plusieurs enzymes différentes, jusqu'à 50, comme par exemple CBHI, CBHII qui interviennent dans l'hydrolyse de la cellulose, et des xylanases qui interviennent dans l'hydrolyse des pentoses. La composition exacte du cocktail dépend de la souche du champignon utilisée et des conditions de culture. Le coût de la solution enzymatique pénalise les procédés de conversion de la biomasse lignocellulosique par voies fermentaires, et des recherches génétiques sont menées sur les champignons afin d'améliorer le cocktail enzymatique. Néanmoins, les mécanismes mis en oeuvre sont peu connus, et les modifications génétiques des champignons sont complexes et délicates. Le principal but de ces travaux est d'améliorer l'hydrolyse enzymatique de la cellulose dans des conditions économiquement raisonnables. Les activités xylanases sont pour l'instant peu étudiées et souvent faibles.

Une possibilité pour limiter le coût global est de réduire la charge enzymatique. Le rendement d'hydrolyse de la cellulose et des hémicelluloses est dépendant des conditions de mise en oeuvre et notamment de la quantité de solution enzymatique ajoutée. Cette dépendance n'est pas linéaire. En effet, une partie des polymères de sucres est facilement hydrolysable suite au prétraitement. De ce fait, une faible dose d'enzymes permettra d'obtenir une meilleure valorisation de la coûteuse solution enzymatique (kg de sucres hydrolysés par kg de solution utilisée). Néanmoins, ceci se fait au détriment du rendement d'hydrolyse des polymères de sucres. Il est important de noter que le coût de la biomasse initiale est aussi conséquent dans le prix de revient du produit final, du fait des rendements massiques limités inhérents aux procédés fermentaires. Par exemple, l'équation stoechiométrique de conversion du glucose en éthanol donne un rendement massique maximum de 0,51 kg d'éthanol par kg de glucose.

A l'inverse, une hydrolyse maximale du substrat nécessitera une dose d'enzymes très importante. Il faut noter que certains prétraitements et/ou substrats produisent des solides prétraités contenant de la cellulose dite récalcitrante qu'il n'est pas possible d'hydrolyser entièrement.

Pour être économiquement viable, un procédé de production d'alcools et/ou solvants de deuxième génération devra se placer sur un compromis entre valorisation de la solution enzymatique et valorisation de la biomasse.

Dans ce type de procédé, l'étape d'hydrolyse enzymatique est suivie d'une fermentation alcoolique. A l'issue de ces deux étapes, on obtient les produits recherchés (alcools et/ou solvants) et des résidus solides contenant encore des polymères de sucres, en quantité plus ou moins importantes selon les performances de l'hydrolyse enzymatique.

Des améliorations dans la valorisation du substrat lignocellulosique aux différentes étapes du procédé de conversion de la biomasse font actuellement l'objet de nombreuses recherches.

La présente invention propose plus particulièrement une valorisation des résidus solides obtenus dans ce type de procédé, en vue d'améliorer le bilan de masse global et donc la viabilité économique du procédé.

### RÉSUMÉ DE L'INVENTION

La présente invention porte sur un procédé de production d'alcools et/ou de solvants dit de seconde génération, dans lequel la biomasse lignocellulosique ou cellulosique subit un prétraitement alcalin.

### DESCRIPTION DES DESSINS

La Figure 1 est une représentation schématique d'un dispositif mettant en oeuvre un procédé de production d'alcools et/ou de solvants, comprenant en outre une étape de recyclage des résidus solides après cuisson acide, selon la présente invention.
La Figure 2 est une représentation schématique d'une mise en oeuvre d'une cuisson acide en plusieurs étapes selon la présente invention.

### DESCRIPTION DÉTAILLÉE DE L'INVENTION

La présente invention décrit un procédé de production d'alcools et/ou de solvants à partir de substrat cellulosique ou lignocellulosique comprenant au moins les étapes suivantes :
- une étape de prétraitement alcalin (étape A) dudit substrat, comprenant une étape de chauffage en présence d'un réactif chimique alcalin (étape A1) et éventuellement une étape de lavage (étape A2), à l'issue desquelles on obtient au moins un substrat prétraité,
- une étape B constituée d'une étape de mise au pH entre 4,5 et 5,5 (étape B1) et d'une étape d'hydrolyse enzymatique (étape B2) du substrat prétraité mettant en jeu des enzymes cellulolytiques et/ou hémicellulolytiques, à l'issue de laquelle on obtient un hydrolysat constitué d'une phase liquide contenant des sucres et d'un résidu solide,
- une étape de fermentation alcoolique (étape C1) de l'hydrolysat obtenu par un microorganisme alcooligène,
- une étape de séparation/purification (étape C2) à l'issue de laquelle on obtient un ou des alcools et/ou solvants purifiés,
- une étape d'extraction du résidu solide,
- une étape de cuisson acide (étape D) d'au moins une fraction du résidu solide extrait, à l'issue de laquelle une partie ou la totalité du produit obtenu est recyclé vers l'étape B.

Le procédé selon la présente invention permet d'améliorer le rendement matière et la valorisation des résidus solides obtenus aux différentes étapes du procédé. Ainsi le bilan économique est plus favorable.

Le produit issu de la cuisson acide, provenant des résidus solides extraits après les différentes étapes, est avantageusement utilisé pour l'étape de mise au pH nécessaire pour réaliser l'hydrolyse enzymatique.

Ainsi, en réalisant l'étape de cuisson acide en conditions peu sévères, les hémicelluloses présents dans les résidus solides insolubles sont hydrolysés en sucres monomères et solubilisés. Peu de produits de dégradation des sucres sont générés et la cellulose présente non digérée encore désignée en tant que "cellulose récalcitrante" devient plus facilement accessible aux enzymes cellulolytiques. Le plus souvent, les cocktails cellulolytiques comme par exemple ceux produits par *T. reesei* ne sont pas suffisamment riches en activités xylanases pour obtenir une hydrolyse efficace des hémicelluloses. Cette hydrolyse acide en conditions douces est une option technologique intéressante, notamment pour les pâtes papetières par exemple issues d'un procédé Kraft et "relativement riches en hémicelluloses" tels que les produits non blanchis.

En réalisant cette cuisson acide en conditions sévères (concentration en acide, durée, température appliquée), il est possible d'hydrolyser chimiquement la totalité ou quasi-totalité de la cellulose récalcitrante à une première hydrolyse enzymatique. Dans ces conditions sévères, les hémicelluloses sont également chimiquement entièrement hydrolysées et d'une part, les sucres pentoses sont pour partie dégradés en furfural, d'autre part, les sucres hexoses sont pour une faible partie dégradés en 5HMF, levulinate et formiate. Par ailleurs, la cuisson acide de la lignine contribue à libérer des produits phénoliques tels que les acides/aldéhydes vanillique, coumarique, férulique, syringique qui tous vont contribuer à protéger la richesse en sucres de l'hydrolysat en rendant le milieu plus toxique pour les contaminants potentiels tels que les bactéries lactiques.

Le prétraitement chimique alcalin réalisé à l'étape A est préférentiellement un prétraitement au sulfate de sodium, encore appelé procédé Kraft, classiquement utilisé dans les procédés de production de produits papetiers, dit Kraft ou "pâte au sulfate", à l'issue duquel on obtient des pâtes papetières.

Le prétraitement chimique alcalin réalisé à l'étape A peut également être un prétraitement par explosion des fibres à l'ammoniac, encore appelé prétraitement AFEX (Ammonia Fiber Explosion) ou prétraitement par percolation utilisant de l'ammoniaque avec recycle, encore appelé prétraitement ARP (Ammonia Recycle Percolation).

L'étape A1 du procédé selon la présente invention est une étape de cuisson en présence d'un réactif chimique alcalin. Ce réactif se présente sous forme liquide ou gazeuse, en fonction du prétraitement mis en oeuvre.

Le procédé au sulfate de sodium ou procédé Kraft est basé sur l'utilisation de soude et de sulfate de sodium. Le traitement chimique des copeaux de bois se fait à 150-175°C pendant une durée de 1 à 7 heures en fonction du substrat utilisé. Les pâtes papetières kraft sont produites à partir des biomasses les plus variées, mais plus particulièrement depuis les espèces arborescentes résineuses (softwood tels que les épicéas ou les pins) ou feuillues (hardwood tels que les eucalyptus) ou encore les déchets lignocellulosiques agricoles (paille de blé, riz, etc.). Elles sont partiellement délignifiées au moyen de cuissons à haute température et en présence de soude. Cette délignification est contrôlée par les paramètres opératoires des réacteurs. La cuisson est réalisée dans un réacteur vertical, où les copeaux descendent par gravité et rencontrent les diverses liqueurs de cuisson. Le sulfure de sodium est préparé directement à partir de sulfate de sodium par combustion. Lors de la cuisson, le sulfure de sodium est hydrolysé en soude, en NaHS et en H2S. Les différents composés soufrés présents réagissent avec la lignine pour donner des thiolignines plus facilement solubles. La liqueur appliquée aux copeaux est appelée liqueur blanche. La liqueur extraite du réacteur ou lessiveur contenant les composés éliminés de la paroi est appelée liqueur noire.

A l'issue de ce prétraitement alcalin, on aboutit à la production d'un substrat prétraité, enrichi en cellulose puisqu'il contient entre 60 et 90% de cellulose et entre 5 et 20% d'hémicellulose.

D'autres prétraitements alcalins sont étudiés à l'échelle du laboratoire, dans le but de réduire les coûts liés à cette étape dans l'optique d'une production de carburant.

Le procédé ARP (Ammonia Recycle Percolation) est un procédé de prétraitement utilisant de l'ammoniaque avec recyclage. Ce type de procédé est notamment décrit par Kim et al., 2003, Biores. Technol. 90 (2003) p 39-47. La température élevée de la percolation conduit à une solubilisation partielle à la fois de la lignine et des hémicelluloses, cette solution est ensuite chauffée pour recycler l'ammoniaque et récupérer d'une part la lignine extraite, par exemple pour une valorisation énergétique, et d'autre part les sucres solubles issus des hémicelluloses.

Le procédé AFEX (Ammonia Fiber Explosion) consiste à introduire le substrat lignocellulosique dans un cuiseur à haute pression en présence d'ammoniac, puis de provoquer une détente explosive en sortie du réacteur et de recycler l'ammoniac alors sous forme gazeuse. Ce type de procédé est notamment décrit par Teymouri et al., 2005, Biores. Technol. 96 (2005) p.2014-2018. Ce procédé conduit principalement à une déstructuration de la matrice de la biomasse mais il n'y a pas de séparation phasique des composés lignine, hémicellulose et cellulose en sortie de traitement.

D'autres traitements alcalins sont aussi à l'étude, notamment à base de soude ou de chaux, une revue non exhaustive est donnée par Ogier et al., 1999, Oil & Gas Science and Technology - Revue de l'IFP, Vol. 54 (1999), No. 1, pp. 67-94.

Ces prétraitements alcalins peuvent être combinés à une action mécanique, créée par exemple dans une extrudeuse de type bi-vis ou une défribreuse.

L'étape A2, optionnelle, est une étape consistant à laver le substrat prétraité par introduction d'un ou plusieurs liquides de lavage. Cette étape de lavage peut également se limiter à une étape de dilution.

L'étape B1 de mise au pH du substrat prétraité, éventuellement lavé, est réalisée par addition d'une solution correctrice de pH.

De façon préférée, la solution correctrice de pH est une solution acide.

L'étape B2 est l'étape d'hydrolyse enzymatique proprement dite, et s'effectue dans des conditions douces , à une température de l'ordre de 45-50°C et pH de 4,5- 5,5 et préférentiellement entre 4,8 et 5,2. Elle est réalisée au moyen d'enzymes produites par un microorganisme. Des microorganismes, comme les champignons appartenant aux genres *Trichoderma, Aspergillus, Penicillium ou Schizophyllum,* ou les bactéries anaérobies appartenant par exemple au genre *Clostridium,* produisent ces enzymes, contenant notamment les cellulases et les hémicellulases, adaptées à l'hydrolyse poussée de la cellulose et des hémicelluloses.

Le micro-organisme peut être produit dans une ligne de production indépendante qui peut être réalisée sur site ou hors site.

De façon très préférée, le micro-organisme utilisé est *Trichoderma reesei.*

Avantageusement, les étapes B1 et B2 sont réalisées simultanément, et la mise au pH se fait dans le réacteur d'hydrolyse enzymatique.

Grâce au prétraitement alcalin réalisé (étape A), la susceptibilité à l'hydrolyse enzymatique est excellente et les polymères de cellulose et d'hémicelluloses sont solubilisés en sucres monomères et/ou oligomères de différents degrés de polymérisation par l'action des enzymes.

Les conditions de l'hydrolyse, principalement le taux de matière sèche du mélange à hydrolyser et la quantité d'enzymes utilisée, conduisent à l'issue de l'étape B à une hydrolyse partielle entre 20% et 90% de la cellulose du substrat prétraité en glucose, et plus particulièrement entre 50% et 80%, ainsi qu'à une hydrolyse partielle entre 20% et 90%, des hémicelluloses solides du substrat prétraité, et plus préférentiellement entre 40% et 70%.

L'étape C1 est l'étape de fermentation par un micro-organisme de l'hydrolysat obtenu après l'hydrolyse enzymatique. Les sucres fermentescibles sont ainsi transformés en alcools et/ou solvants par des levures ou autres micro-organismes. La fermentation est réalisée le plus souvent à une température comprise entre 30 et 35°C. A l'issue de cette étape, on obtient un moût de fermentation comprenant des matières en suspension et une phase liquide dans laquelle se trouve le ou les produits recherchés (alcools et/ou solvants).

Lorsque les étapes d'hydrolyse enzymatique B2 et de fermentation alcoolique C1 sont réalisées simultanément dans un même réacteur, on parle de procédé SSF. La température est alors située autour de 35°C.

Lorsqu'on réalise simultanément l'hydrolyse enzymatique des polymères de sucres et la fermentation du glucose et xylose, on parle de procédé SSCF (Simultaneous Saccharifiaction and Co Fermentation). La température est alors située autour de 35°C.

L'étape C2 est une étape de séparation/purification du produit qui permet d'obtenir un ou des alcools et/ou solvants purifiés prêt à être vendus. Cette étape comprend avantageusement au moins deux sous-étapes : une étape de distillation et une étape de déshydratation.

Chacune de ces différentes étapes conduisent à la production d'une phase liquide, en mélange avec des résidus solides insolubles.

Le procédé selon la présente invention propose donc de valoriser les résidus solides. Il est donc nécessaire de les séparer des phases liquides. Cette séparation se fait lors de l'étape d'extraction, notamment par pressage, centrifugation ou filtration, ainsi que par toute autre technique connue de l'homme du métier. Les limites actuelles des équipements de séparation solide/liquide conduisent à la présence d'une partie de la phase liquide dans le gâteau extrait, entre 25 et 80% poids.

L'étape d'extraction du résidu solide peut être réalisée après l'étape d'hydrolyse enzymatique B2 et avant l'étape C1 de fermentation. Dans ce cas, le gâteau extrait comprend d'une part une phase liquide comprenant entre 75 et 99% poids d'eau, et des sucres et d'autre part des résidus solides, comprenant de la cellulose non-hydrolysée, dont une partie peut être récalcitrante, des hémicelluloses non hydrolysées, de la lignine et autres composés insolubles initialement présents dans le substrat.

Selon un autre mode de réalisation du procédé selon l'invention, l'étape d'extraction du résidu solide peut être réalisée entre l'étape de fermentation (étape C1) et l'étape de séparation/purification (étape C2). Dans ce cas, le gâteau extrait comprend d'une part une phase liquide comprenant entre 75% et 99%poids d'eau, des alcools et/ou solvants (produits) et des sucres non-convertis; et d'autre part, des résidus solides, comprenant de la cellulose non-hydrolysée, dont une partie peut être récalcitrante, des hémicelluloses non hydrolysées, de la lignine et autres composés insolubles initialement présents dans le substrat; ainsi qu'une quantité de micro-organismes alcooligènes (comprise entre 2% et 20% en fonction des performances de l'hydrolyse enzymatique et de la fermentation alcoolique).

Selon un autre mode de réalisation, l'étape d'extraction du résidu solide est réalisée après l'étape de séparation/purification (étape C2). Dans ce cas, le gâteau extrait comprend d'une part une phase liquide comprenant entre 80 et 99%poids d'eau, des sucres non-convertis et des traces de produits (moins de 0,1%); et d'autre part des résidus solides, comprenant de la cellulose non-hydrolysée, dont une partie peut être récalcitrante, des hémicelluloses non hydrolysées, de la lignine et autres composés insolubles initialement présents dans le substrat; ainsi qu'une faible quantité de micro-organismes alcooligènes (comprise entre 2% et 20% en fonction des performances de l'hydrolyse enzymatique et de la fermentation alcoolique). La proportion de chaque composant dans ce résidu solide est fonction du substrat initial, du type de prétraitement alcalin réalisé et des conditions de mise en oeuvre de l'hydrolyse enzymatique

La proportion de chaque composant dans le résidu solide extrait est fonction du substrat initial, du type de prétraitement alcalin réalisé et des conditions de mise en oeuvre de l'hydrolyse enzymatique.

Dans le cas d'un prétraitement délignifiant de type Kraft, la quantité de lignine est comprise entre 2 et 40% poids, et plus préférentiellement entre 3% et 30% poids, la quantité de cellulose entre 15 et 85% poids, préférentiellement entre 25% et 82% poids et la quantité d'hémicelluloses entre 3 et 60% poids, et préférentiellement entre 5 et 50% poids.

Dans le cas d'un prétraitement partiellement délignifiant, de type ARP, la quantité de lignine est comprise entre 4 et 65% poids, et plus préférentiellement entre 9% et 50% poids, la quantité de cellulose entre 12 et 80% poids, préférentiellement entre 20% et 70% poids et la quantité d'hémicelluloses entre 3 et 60% poids, et préférentiellement entre 5% et 45% poids.

Dans le cas d'un prétraitement alcalin non délignifiant, comme par exemple l'AFEX, la quantité de lignine est comprise entre 15 et 70%, et plus préférentiellement entre 20% et 55%, la quantité de cellulose entre 4 et 50%, préférentiellement entre 10 et 45% et la quantité d'hémicelluloses entre 5 et 50%, et préférentiellement entre 12 et 40%.

Avantageusement, un lavage du gâteau peut-être réalisé afin de récupérer les sucres et/ou produits piégés dans la phase liquide. La phase liquide du gâteau obtenue après lavage contient essentiellement de l'eau, et la phase solide n'est pas ou peu modifiée.

Au cours de l'étape D, une fraction des résidus solides subit une cuisson acide, en présence d'une solution acide comprenant un acide fort de préférence choisi parmi l'acide sulfurique ou phosphorique, pendant un temps compris entre 10 minutes et 3 heures, à une température comprise entre 105 et 240°C, avec un ratio acide /matière solide (MS) compris entre 0,1 et 100%, préférentiellement entre 0,5 et 70% et encore plus préférentiellement entre 0,25 et 30%.

Selon un mode de réalisation du procédé selon l'invention, l'acide utilisé lors de l'étape D est une fraction de la solution correctrice de pH utilisée lors de l'étape B1.

La fraction de résidus solides envoyés à l'étape D représente entre 15 et 100% poids de la quantité totale de résidus solides extraits, préférentiellement entre 20 et 90% poids et encore plus préférentiellement entre 50 et 80% poids.

L'étape de cuisson acide est réalisée en une ou plusieurs étapes.

Dans un premier mode de réalisation l'étape D de cuisson acide est réalisée en une seule étape.

A l'issue de cette étape, plus de 20% poids, de préférence plus de 70% poids et encore plus préférentiellement plus de 90% poids des hémicelluloses contenus dans les résidus solides sont hydrolysées. Une faible proportion correspondant à moins de 10% poids, préférentiellement moins de 5 % poids et encore plus préférentiellement moins de 2% poids est dégradée.

Une fraction de la cellulose est aussi hydrolysée, mais en quantité moins importante que la fraction hémicellulose. De préférence moins de 20% poids de la cellulose, et de préférence moins de 10%, et encore plus préférentiellement entre 2 et de 3% poids est dégradée.

Selon un autre mode de réalisation du procédé selon la présente invention, l'étape D de cuisson acide est réalisée en plusieurs étapes successives de cuisson. Il est alors possible de faire varier les conditions de cuisson pour chacune des étapes. Au cours d'une de ces étapes, il est possible de choisir des conditions de cuisson acides telles qu'elles permettent une hydrolyse poussée de la cellulose récalcitrante à l'hydrolyse enzymatique.

Lorsque l'étape de cuisson acide est réalisée en plusieurs étapes, entre chaque étape de cuisson, on réalise avantageusement une étape de séparation, pour séparer la phase liquide du résidu solide. Chaque fraction liquide séparée est alors recyclée vers l'étape B.

Le produit issu de la cuisson acide (étape D) est recyclé au moins en partie vers l'étape B. Avantageusement, il est utilisé pour participer à l'étape de mise au pH (étape B1) nécessaire pour réaliser l'hydrolyse enzymatique.

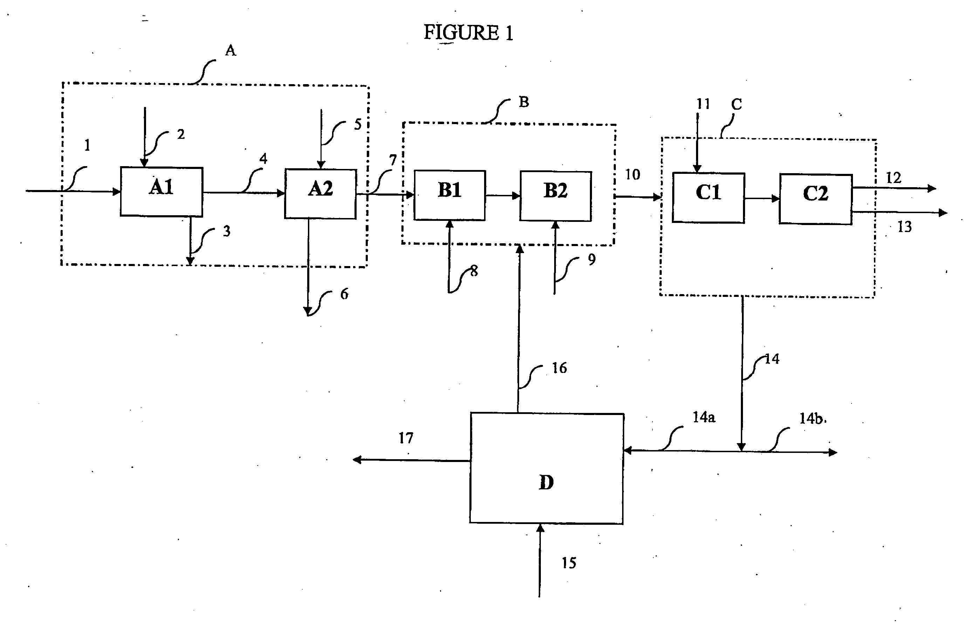

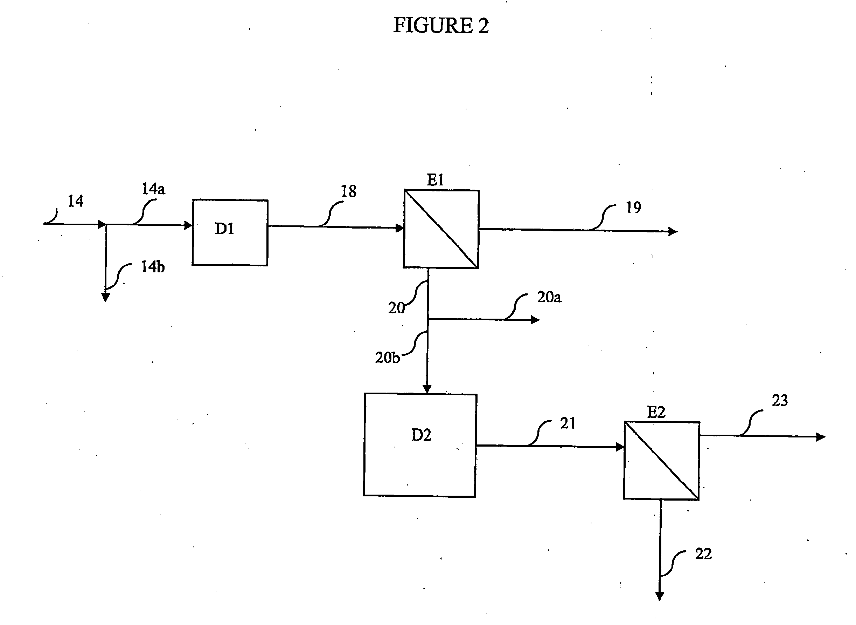

L'invention va être décrite en se référant aux Figures 1 et 2.

Les postes A à D représentés sur les figures correspondent respectivement aux différentes étapes du procédé selon la présente invention, désignées par les mêmes lettres.

Le fonctionnement du poste A est dépendant du prétraitement alcalin réalisé.

Dans le cas d'un prétraitement dit Kraft, la biomasse est introduite par une conduite 1 dans le réacteur de cuisson ou lessiveur (A1). Une solution alcaline, encore appelée liqueur blanche, y est également introduite via la conduite 2. La biomasse est partiellement délignifiée au moyen de cuissons à haute température et en présence de soude. La lignine solubilisée est retirée avec la solution alcaline et est évacuée par la conduite 3 avec la solution alcaline usée, aussi appelée liqueur noire.

Cette étape de délignification peut avoir lieu dans plusieurs lessiveurs successifs non représentés sur les figures et est contrôlée par les paramètres opératoires fixés dans ces dispositifs.

La pâte obtenue en sortie des lessiveurs circulant dans la conduite 4 est enrichie en cellulose : elle contient entre 60 et 90 % en poids de cellulose par rapport à la matière solide totale. Elle est envoyée vers le réacteur A2 pour subir une étape de lavage : un ou plusieurs liquides de lavage peuvent être introduits via une conduite 5. Les liquides de lavage usés sont retirés par la conduite 6.

Dans le cas d'un traitement AFEX, la biomasse est introduite par la conduite 1 dans un réacteur de cuisson (A1). Une solution ammoniacale est introduite par la conduite 2 sous pression, de 15 à 30 bars, à température modérée (70°C à 110°C). Le mélange est maintenu dans ces conditions durant un temps déterminé en fonction du substrat, puis détendu en sortie du cuiseur. La solution ammoniacale est récupérée via la conduite 3 sous forme gazeuse pour être recyclée. Le substrat prétraité extrait par la conduite 4 du cuiseur a essentiellement la même composition que le substrat en entrée. L'étape de lavage A2 après ce prétraitement peut se limiter à une étape de dilution avec un liquide de dilution introduit par la conduite 5, auquel cas le flux de liquide de lavage évacué par la conduite 6 est nul.

Dans le cas d'un prétraitement ARP, la biomasse est introduite par la conduite 1 dans un réacteur de cuisson (A1). Une solution ammoniacale est percolée sur la biomasse sous pression (15 à 30 bars) et à température élevée, 130°C à 190°C. La biomasse est partiellement délignifiée, une partie des hémicelluloses est aussi solubilisée. Les sucres et la lignine solubilisés sont retirés avec la solution alcaline usée et sont évacués par la conduite 3. Le substrat issu de la cuisson et extrait par la conduite 4 est lavé dans un réacteur de lavage (A2): un ou plusieurs liquides de lavage peuvent être introduits par la conduite 5. Les liquides de lavage usés sont retirés via la conduite 6.

Le substrat prétraité, éventuellement lavé, ainsi obtenu circulant dans la conduite 7 comprend entre 50 et 95% poids de matières insolubles dans l'eau, et plus particulièrement entre 60 et 85% poids de matières insolubles dans l'eau.

L'étape de mise au pH est réalisée au niveau du poste B1, par introduction, via la conduite 8, d'une solution correctrice de pH.

Le pH doit être ajusté jusqu'à obtenir le pH optimal entre 4,5 et 5,5, préférentiellement entre 4,8 et 5,2 pour l'étape d'hydrolyse enzymatique.

L'hydrolyse enzymatique est réalisée sur un substrat prétraité qui contient entre 1% et 50% poids de matière sèche (insoluble dans un premier temps, puis solubilisée par action enzymatique), de préférence entre 7% et 30%, et plus préférentiellement entre 10% et 25%.

Les enzymes nécessaires pour cette étape B2 sont introduites par la conduite 9. L'hydrolysat obtenu après l'hydrolyse enzymatique est introduit dans le réacteur de fermentation C1 par la conduite 10. La fermentation est assurée par des levures ou autres microorganismes, introduits dans le réacteur par la conduite 11.

L'étape C1 est suivie d'une étape de séparation/purification C2 à l'issue de laquelle on obtient le ou les produits recherchés (alcools et/solvants) extraits par la conduite 12, et des vinasses extraites par la conduite 13.

Le résidu solide obtenu après les étapes précédentes est évacué par la conduite 14.

Sur la Figure 1, cette conduite est représentée de façon non limitative comme provenant du poste C.

Une fraction 14a du résidu solide est envoyée vers le réacteur D de cuisson acide, dans lequel une solution acide est introduite par la conduite 15.

La fraction 14b qui n'est pas envoyée dans le réacteur de cuisson acide est extraite en vue de la valorisation de sa matière, par exemple vers une valorisation énergétique par combustion.

Une fraction du produit obtenu après la cuisson acide est envoyée par la conduite 16 vers le poste B, où sont réalisées la mise au pH et l'hydrolyse enzymatique. Cette fraction représente moins de 60% poids et de préférence moins de 35% poids. L'autre fraction, identique ou éventuellement modifiée par une opération de séparation solide/ liquide, est évacuée par la conduite 17.

Selon un mode particulier de réalisation, la totalité du produit obtenu après l'étape de cuisson acide est envoyée vers le poste B.

Grâce au procédé selon la présente invention, la susceptibilité à l'hydrolyse enzymatique de la phase solide du produit acide circulant dans la conduite 16 est supérieure ou égale à celle du résidu 14 avant la cuisson acide.

Selon le mode de réalisation représenté sur Figure 2, la cuisson acide peut être avantageusement réalisée en plusieurs étapes.

Une fraction 14a du résidu solide est envoyée vers un premier réacteur de cuisson D1, opérant dans des conditions douces. Le produit obtenu est évacué par la conduite 18 vers un séparateur E1. En sortie du séparateur, on extrait une phase liquide comprenant des hémicelluloses hydrolysées par la conduite 19, qui peut être recyclée et renvoyée vers le poste B et un résidu solide contenant de la cellulose évacué par la conduite 20.

Avantageusement, une partie du résidu solide est extrait directement par la conduite 20a car la cellulose de cette fraction insoluble a une digestibilité enzymatique retrouvée voire nettement améliorée et elle est donc plus accessible aux enzymes cellulolytiques.

L'autre partie 20b est envoyée vers un deuxième réacteur de cuisson, opérant dans des conditions plus sévères que le réacteur D1, pour réaliser une hydrolyse poussée de la cellulose

Le produit obtenu après cette deuxième étape de cuisson acide D2 est envoyé par une conduite 21 vers un deuxième séparateur E2. On extrait ainsi, en sortie de E2 par la conduite 23, une phase liquide qui peut être avantageusement recyclée vers le poste B et un résidu solide extrait par la conduite 22 très pauvre en cellulose. Comme déjà mentionné, cette cellulose insoluble, en faible proportion, est plus accessible aux enzymes cellulolytiques et peut également être recyclée vers le poste B.

Ainsi, en réalisant une cuisson acide en plusieurs étapes, il devient possible d'extraire une quantité nettement améliorée de cellulose des résidus solides, et par conséquent d'améliorer leur valorisation.

Le recyclage des polysaccharides non hydrolysés permet d'augmenter le rendement poids total du procédé. Avantageusement, ce gain en rendement peut être réalisé sans augmenter la quantité ou le débit de produits chimiques mis en oeuvre, tout particulièrement les acides. Enfin, l'hydrolyse plus poussée des pentoses du gâteau permet de compenser le cas échéant la moindre hydrolyse enzymatique des pentoses, et donc d'éviter l'accumulation des pentoses dans le gâteau qui peut apparaître dans un recyclage classique.

### EXEMPLES

### Exemple 1 (non conforme à l'invention) :

On considère un procédé de production d'éthanol à partir de bois, où le bois est prétraité dans un procédé papetier type Kraft, qui est un procédé alcalin en présence de soude. La pâte pauvre en lignine issue du Kraft est ensuite lavée et neutralisée, puis introduite dans le procédé de conversion de substrat cellulosique en éthanol par hydrolyse enzymatique et fermentation du glucose.

Le procédé traite 100 tonnes / heure de bois à 20% d'humidité. La composition de la matière sèche est comme suit:

| | |
|---|---|
| Cellulose | 46.3% |
| Hémicelluloses | 17.0% |
| Acétate | 0.7% |
| Lignine | 29.0% |
| Cendres | 3.5% |
| Autres | 3.5% |

Le procédé Kraft est opéré dans des conditions telles que la pâte papetière en sortie conserve:

| | |
|---|---|
| 98.0% | de la Cellulose Initiale |
| 65.0% | des Hémicelluloses initiales |
| 100.0% | de l'Acétate initial |
| 5.0% | de la Lignine initiale |
| 80.0% | des Cendres initiales |
| 80.0% | des Autres composés initiaux |

Le débit de pâte papetière en sortie du Kraft est ainsi de 51.3 tonnes / heure de matière sèche.

Le procédé de lavage est tel que la pâte lavée a un pH de 9,0 et contient 13% de matière solide.

Le procédé de conversion en éthanol contient les étapes suivantes : hydrolyse enzymatique, fermentation alcoolique, séparation des résidus solides, distillation et déshydratation de l'éthanol. L'hydrolyse enzymatique s'opère à pH 4,8. De ce fait, la consommation d'acide 8 pour la rectification du pH de la pulpe papetière est de 8 880N. Dans les conditions d'hydrolyse choisies, le rendement de l'hydrolyse enzymatique de la cellulose est de 65%, alors que celui des hémicelluloses est de 35%, et le rendement de fermentation est 95% du maximum théorique. Le procédé produit 12,27 tonnes / heure d'éthanol à 99.7%, et 54,67 tonnes *l* heure de gâteau solide à 40% de matière solide.

### Exemple 2 (selon l'invention)

On décrit ici la mise en oeuvre de l'invention dans le procédé décrit dans l'exemple 1. Ainsi, 50 tonnes / heure du gâteau produit sont envoyés dans un réacteur de cuisson acide. La cuisson acide s'effectue à 150°C pendant une heure en présence de H₂SO₄. La quantité mise en oeuvre ici est de 20g de H₂SO₄ / kg de matière solide (2%poids). L'hydrolyse acide conduit à une conversion de toutes les hémicelluloses en sucres C5, avec une dégradation de 3% en furfural. 90% des sucres C5 et C6 présents dans le gâteau en début d'hydrolyse acide sont convertis respectivement en furfural et 5-hydromethylfurfural. Le substrat issu de l'hydrolyse acide est mélangé à la pulpe lavée avant l'étape de neutralisation, apportant ainsi 8 412N, l'apport complémentaire d'acide pour ramener le mélange au pH de l'hydrolyse est de 468N. Les rendements du procédé de conversion du substrat en éthanol sont conservés, la production d'éthanol est donc de 15,95 tonnes / heure, soit une augmentation du rendement global du procédé de 30%, pour une même consommation de bois (80 tonnes / heure de matière sèche) et d'acide (8 880 N).

### Exemple 3 (non conforme à l'invention)

On considère un procédé de production d'un mélange acétone-butanol-éthanol à partir de paille.

La paille est conditionnée puis traitée dans un procédé dit AFEX, qui consiste en une imprégnation de la paille à l'ammoniac sous pression, suivie d'une détente explosive. Le substrat est ensuite lavé puis neutralisé, avant d'être introduit dans un procédé de production d'acétone-butanol-éthanol par hydrolyse enzymatique, séparation du résidu solide puis fermentation des sucres issus de la cellulose et des hémicelluloses (C5 et C6).

Le procédé traite 150 tonnes / heure de paille à 15% d'humidité. La composition de la matière sèche est comme suit:

| | |
|---|---|
| Cellulose | 40.0% |
| Hémicelluloses | 27.0% |
| Acétate | 0.7% |
| Lignine | 23.0% |
| Cendres | 6.0% |
| Autres | 3.3% |

Le procédé AFEX est opéré dans des conditions telles que le substrat en sortie a la même composition et le même débit que le substrat en entrée. Tout l'ammoniac est extrait du substrat. Le substrat a donc un pH neutre à l'issue du prétraitement. Le substrat est ensuite mis en suspension avant d'être soumis à l'hydrolyse enzymatique, le pH de la solution étant ajusté à pH=5 pour cette étape, ce qui entraîne la consommation de 9350 N d'acide. L'hydrolyse est opérée dans des conditions qui permettent d'obtenir 85% d'hydrolyse de la cellulose et 70% des hemicelluloses.

Après la séparation du solide résiduel, 53,5 tonnes / heure de matière solide, la fermentation des sucres permet de produire 33g de mélange ABE par 100 g de sucres (glucose + xylose), soit un débit de 22,2 tonnes / heure d'ABE.

### Exemple 4 (selon l'invention)

On décrit ici la mise en oeuvre de l'invention dans le procédé décrit dans l'exemple 3.

Ainsi, 16,5 tonnes de matière solide / heure du gâteau produit sont envoyées dans un réacteur de cuisson acide. La quantité est limitée pour maintenir 25% de cellulose+hémicellulose dans la composition du gâteau. La cuisson acide s'effectue à 150°C pendant 40 minutes en présence de H₂SO₄. La quantité mise en oeuvre ici est de 36g de H₂SO₄ / kg de matière solide (3,6%poids). L'hydrolyse acide conduit à une conversion de 85% des hémicelluloses en sucres C5, plus une dégradation de 7% en furfural. 70% des sucres C5 et C6 présents dans le gâteau en début d'hydrolyse acide sont convertis respectivement en furfural et 5-hydromethylfurfural. Le substrat issu de l'hydrolyse acide est mélangé à la pulpe lavée avant l'étape de neutralisation, apportant ainsi 9 183N, l'apport complémentaire d'acide pour ramener le mélange au pH de l'hydrolyse est de 167N.

Les rendements du procédé de conversion du substrat en ABE sont conservés, la production d'ABE est ainsi de 23,7 tonnes / heure, soit une augmentation du rendement global du procédé de 7%, pour une même consommation de paille (150 tonnes / heure de matière sèche) et d'acide (9 350N).

### Exemple 5 (selon l'invention)

On décrit ici la mise en oeuvre d'une variante de l'invention dans le procédé décrit dans l'exemple 3.

Ainsi, 85% du gâteau produit est envoyé dans un procédé de cuisson acide en deux étapes. La première cuisson s'effectue à 135°C pendant 90 minutes en présence de H₂SO₄, puis une séparation solide-liquide est effectuée. La seconde cuisson s'effectue à 200°C pendant 80 minutes. La quantité mise en oeuvre est de 20g de H₂SO₄ / kg de matière solide (2%poids) pendant la première cuisson et de 150g de H₂SO₄ / kg de matière solide (15%poids) pendant la seconde. La première étape d'hydrolyse acide conduit à une conversion de 85% des hémicelluloses en sucres C5, plus une dégradation de 2% en furfural, la seconde étape convertit 80% de la cellulose en glucose. 75% des sucres C5 et C6 présents dans le gâteau en début d'hydrolyse acide sont convertis respectivement en furfural et 5-hydromethylfurfural et en produits de dégradation de ce dernier. Le substrat liquide issu des étapes d'hydrolyse acide est mélangé à la pulpe lavée avant l'étape de neutralisation, apportant ainsi 115 310N. Un correcteur de pH basique (105 960N) est mélangé à ce flux pour ramener le mélange au pH de l'hydrolyse.

La présence de glucose dans le substrat en entrée d'hydrolyse enzymatique diminue quelque peu le rendement de cette hydrolyse du fait de l'inhibition par leur produit des enzymes, ce rendement est donc de 80% (et non 85% dans les mêmes conditions en absence de glucose).

Les rendements de fermentation des sucres en ABE sont conservés, la production d'ABE est ainsi de 25,5 tonnes / heure, soit une augmentation du rendement global du procédé de 15%, pour une même consommation de paille (150 tonnes / heure de matière sèche).

## Revendications

1. Procédé de production d'alcools et/ou de solvants à partir d'un substrat cellulosique ou lignocellulosique comprenant au moins les étapes suivantes :
- une étape de prétraitement alcalin (étape A) dudit substrat, comprenant une étape de chauffage en présence d'un réactif chimique alcalin (étape A1) et éventuellement une étape de lavage (étape A2), à l'issue desquelles on obtient au moins un substrat prétraité ,
- une étape B constituée d'une étape de mise au pH entre 4,5 et 5,5 (étape B1) et d'une étape d'hydrolyse enzymatique (étape B2) du substrat prétraité mettant en jeu des enzymes cellulolytiques et/ou hémicellulolytiques, à l'issue de laquelle on obtient un hydrolysat constitué d'une phase liquide contenant des sucres et d'un résidu solide,
- une étape de fermentation alcoolique (étape C1) de l'hydrolysat obtenu par un microorganisme alcooligène,
- une étape de séparation/purification (étape C2) à l'issue de laquelle on obtient un ou des alcools et/ou solvants purifiés,
- une étape d'extraction du résidu solide,
- une étape de cuisson acide (étape D) d'au moins une fraction du résidu solide extrait, à l'issue de laquelle une partie ou la totalité du produit obtenu est recyclé vers l'étape B.

2. Procédé selon la revendication 1 dans lequel le prétraitement chimique réalisé à l'étape A est un prétraitement au sulfate de sodium, encore appelé procédé Kraft à l'issue duquel on obtient des pâtes papetières.

3. Procédé selon la revendication 1 dans lequel le prétraitement chimique réalisé à l'étape A est un prétraitement par explosion des fibres à l'ammoniac, encore appelé prétraitement AFEX ou un prétraitement par percolation utilisant de l'ammoniaque avec recycle, encore appelé prétraitement ARP.

4. Procédé selon l'une des revendications précédentes dans lequel l'étape de mise au pH et l'étape d'hydrolyse enzymatique sont réalisées simultanément, dans le même réacteur.

5. Procédé selon l'une des revendications précédentes dans lequel l'étape d'extraction du résidu solide est réalisée après l'étape d'hydrolyse enzymatique B2 et avant l'étape C1 de fermentation.

6. Procédé selon l'une des revendications 1 à 4 dans lequel l'étape d'extraction du résidu solide est réalisée entre l'étape de fermentation C1 et l'étape C2 de séparation/purification.

7. Procédé selon l'une des revendications 1 à 4 dans lequel l'étape d'extraction du résidu solide est réalisée après l'étape de séparation/purification.

8. Procédé selon l'une des revendications précédentes dans lequel la fraction de résidu solide envoyée à l'étape de cuisson acide représente entre 15 et 100% poids de la quantité totale de résidus solides extraits, préférentiellement entre 20 et 90% poids et encore plus préférentiellement entre 50 et 80% poids.

9. Procédé selon l'une des revendications précédentes dans lequel l'étape de cuisson acide est réalisée à une température comprise entre 105°C et 240°C pendant une durée comprise entre 10 minutes et 3 heures en présence d'une solution acide comprenant un acide fort préférentiellement choisi parmi l'acide sulfurique ou l'acide phosphorique, avec un ratio acide / matière solide(MS) compris entre 0,1 et 100%.

10. Procédé selon l'une des revendications précédentes dans lequel l'étape de cuisson acide est réalisée en une ou plusieurs étapes.

11. Procédé selon la revendication 10 dans lequel l'étape de cuisson acide est réalisée en plusieurs étapes, une étape de séparation d'une fraction liquide et de résidus solides étant réalisée entre chaque étape de cuisson acide.

12. Procédé selon la revendication 11 dans lequel chaque fraction liquide séparée est recyclée vers l'étape B.

## Patentansprüche

1. Verfahren zur Herstellung von Alkoholen und/oder Lösemitteln aus einem Cellulose- oder Lignocellulosesubstrat, umfassend mindestens die folgenden Schritte:
- einen Schritt zur alkalischen Vorbehandlung (Schritt A) des Substrats, umfassend einen Schritt zum Erhitzen in Gegenwart eines chemischen, alkalischen Reaktionsmittels (Schritt A1) und gegebenenfalls einen Schritt zum Waschen (Schritt A2), an deren Ende mindestens ein vorbehandeltes Substrat erhalten wird,
- einen Schritt B, der aus einem Schritt zum Einstellen des pH-Werts auf zwischen 4,5 und 5,5 (Schritt B1) und einem Schritt zur enzymatischen Hydrolyse (Schritt B2) des vorbehandelten Substrats besteht, bei dem cellulosespaltende und/oder hemicellulosespaltende Enzyme eingesetzt werden, an dessen Ende ein Hydrolysat erhalten wird, das aus einer flüssigen Phase besteht, die Zucker und einen festen Rückstand enthält,
- einen Schritt zur alkoholischen Fermentation (Schritt C1) des Hydrolysats, erhalten durch einen alkoholliefernden Mikroorganismus,
- einen Schritt zur Trennung/Reinigung (Schritt C2), an dessen Ende ein oder mehrere gereinigte Alkohole und/oder Lösemittel erhalten werden,
- einen Schritt zur Extraktion des festen Rückstands,
- einen Schritt zum Kochen in Säure (Schritt D) mindestens einer Fraktion des extrahierten festen Rückstands, an dessen Ende ein Teil oder die Gesamtheit des erhaltenen Produkts zu Schritt B recycelt wird.

2. Verfahren nach Anspruch 1, wobei die chemische Vorbehandlung, die in Schritt A durchgeführt wird, eine Vorbehandlung mit Natriumsulfat, auch Kraft-Verfahren genannt, ist, an dessen Ende Papierpulpen erhalten werden.

3. Verfahren nach Anspruch 1, wobei die chemische Vorbehandlung, die in Schritt A durchgeführt wird, eine Vorbehandlung durch Explosion der Fasern mit Ammoniak, auch AFEX-Vorbehandlung genannt, oder eine Vorbehandlung durch Perkolation, wobei flüssiger Ammoniak, der recycelt werden kann, verwendet wird, auch ARP-Vorbehandlung genannt, ist.

4. Verfahren nach einem der vorhergehenden Ansprüche, wobei der Schritt zum Einstellen des pH-Werts und der Schritt zur enzymatischen Hydrolyse gleichzeitig in dem gleichen Reaktor durchgeführt werden.

5. Verfahren nach einem der vorhergehenden Ansprüche, wobei der Schritt zur Extraktion des festen Rückstands nach dem Schritt zur enzymatischen Hydrolyse B2 und vor dem Schritt C1 zur Fermentation durchgeführt wird.

6. Verfahren nach einem der Ansprüche 1 bis 4, wobei der Schritt zur Extraktion des festen Rückstands zwischen dem Schritt zur Fermentation C1 und dem Schritt C2 zur Trennung/Reinigung durchgeführt wird.

7. Verfahren nach einem der Ansprüche 1 bis 4, wobei der Schritt zur Extraktion des festen Rückstands nach dem Schritt zur Trennung/Reinigung durchgeführt wird.

8. Verfahren nach einem der vorhergehenden Ansprüche, wobei die Fraktion des festen Rückstands, die in den Schritt zum Kochen in Säure geschickt wird, zwischen 15 und 100 Gew.-%, vorzugsweise zwischen 20 und 90 Gew.-% und noch stärker bevorzugt zwischen 50 und 80 Gew.-% der Gesamtmenge an extrahierten festen Rückständen darstellt.

9. Verfahren nach einem der vorhergehenden Ansprüche, wobei der Schritt zum Kochen in Säure bei einer Temperatur im Bereich zwischen 105 °C und 240 °C während einer Dauer im Bereich zwischen 10 Minuten und 3 Stunden in Gegenwart einer sauren Lösung durchgeführt wird, die eine starke Säure umfasst, bevorzugt ausgewählt aus Schwefelsäure oder Phosphorsäure, mit einem Verhältnis Säure/Feststoff (MS) im Bereich zwischen 0,1 und 100 %.

10. Verfahren nach einem der vorhergehenden Ansprüche, wobei der Schritt zum Kochen in Säure in einem oder mehreren Schritten durchgeführt wird.

11. Verfahren nach Anspruch 10, wobei der Schritt zum Kochen in Säure in mehreren Schritten durchgeführt wird, wobei zwischen jedem Schritt zum Kochen in Säure ein Schritt zum Trennen einer flüssigen Fraktion von festen Rückständen durchgeführt wird.

12. Verfahren nach Anspruch 11, wobei jede getrennte flüssige Fraktion zu Schritt B recycelt wird.

## Claims

1. A process for the production of alcohols and/or solvents from a cellulosic or lignocellulosic substrate, comprising at least the following steps:
• a step for alkaline pre-treatment (step A) of said substrate, comprising a step for heating in the presence of an alkaline chemical reagent (step A1) and an optional washing step (step A2), at the end of which at least one pre-treated substrate is obtained;
• a step B constituted by a step for adjusting the pH to between 4.5 and 5.5 (step B1) and a step for enzymatic hydrolysis (step B2) of the pre-treated substrate using cellulolytic and/or hemicellulolytic enzymes, at the end of which a hydrolysate is obtained constituted by a liquid phase containing sugars and a solid residue;
• a step for alcoholic fermentation (step C1) of the hydrolysate obtained by an alcohol-producing microorganism;
• a step for separation/purification (step C2) at the end of which one or more purified alcohols and/or solvents are obtained;
• a step for extracting the solid residue;
• a step for acid digestion (step D) of at least a fraction of the extracted solid residue, at the end of which a portion or all of the product obtained is recycled to step B.

2. A process according to claim 1, in which the chemical pre-treatment carried out in step A is a sodium sulphate pre-treatment, also known as the Kraft process, at the end of which paper pulp is obtained.

3. A process according to claim 1, in which the chemical pre-treatment carried out in step A is an ammonia fiber explosion pre-treatment, also termed AFEX pre-treatment, or an ammonia recycle percolation pre-treatment, also termed ARP pre-treatment.

4. A process according to one of the preceding claims, in which the step for adjusting the pH and the step for enzymatic hydrolysis are carried out simultaneously in the same reactor.

5. A process according to one of the preceding claims, in which the step for extracting the solid residue is carried out after the enzymatic hydrolysis step B2 and before the fermentation step C1.

6. A process according to one of claims 1 to 4, in which the step for extracting the solid residue is carried out between the fermentation step C1 and the step C2 for separation/purification.

7. A process according to one of claims 1 to 4, in which the step for extracting the solid residue is carried out after the separation/purification step.

8. A process according to one of the preceding claims, in which the fraction of solid residue sent to the acid digestion step represents in the range 15% to 100% by weight of the total quantity of extracted solid residues, preferably in the range 20% to 90% by weight and more preferably in the range 50% to 80% by weight.

9. A process according to one of the preceding claims, in which the acid digestion step is carried out at a temperature in the range 105°C to 240°C for a period in the range 10 minutes to 3 hours in the presence of an acid solution comprising a strong acid, preferably selected from sulphuric acid or phosphoric acid, with an acid/solid material (SM) ratio in the range 0.1% to 100%.

10. A process according to one of the preceding claims, in which the acid digestion step is carried out in one or more steps.

11. A process according to claim 10, in which the acid digestion step is carried out in several steps, a step for separating a liquid fraction and solid residues being carried out between each acid digestion step.

12. A process according to claim 11, in which each separated liquid fraction is recycled to step B.
